# EUROPEAN PATENT APPLICATION

(11) **EP 4 445 875 A1**
(43) Date of publication of application: **16.10.2024**
(21) Application number: 24168863.9
(22) Date of filing: 05.04.2024
(51) Int. Cl.: A61F 2/34, A61F 2/40

(54) **BONE IMPLANT SHELL WITH MULTIPLE FIXATION OPTIONS**

(30) Priority: 07.04.2023 US 202363457885 P
(71) Applicant: Howmedica Osteonics Corporation, Mahwah, New Jersey 07430 (US)
(72) Inventor: HAGGERTY, Zz, MONTCLAIR, 07042 (US); ROY, Shammodip, WAYNE, 07470 (US); SLAYTON, Alexander, DENVILLE, 07834 (US); MULLEN, Lewis, MAHWAH, 07430 (US); WOOD, Richard, MARLBORO, 07746 (US); FERKO, Michael, WARWICK, 10990 (US); TULKIS, Peter, PARAMUS, 07652 (US)
(74) Representative: Regimbeau

(57) **Abstract**

An orthopedic implant (100, 400) includes a first surface (106), a second surface (108), and a first body portion (109) extending between the first surface (106) and the second surface (108) to define a thickness of the implant (100, 400). The first body portion (109) includes a first porous section (412a-412c) defining pores, solid bounding struts (432a-432i, 434a-434d) including a first set of bounding struts (432a-432i, 434a-434d), and a first bone screw hole (436a-436c) extending through a thickness of the implant (100, 400). Each of the struts of the first set of bounding struts (432a-432i, 434a-434d) bound the first porous section (412a-412c). The first set of bounding struts (432a-432i, 434a-434d) include a first curved strut (432a-432i) that further bounds a portion of a perimeter of the first bone screw hole (436a-436c).

## Description

### CROSS-REFERENCE TO RELATED APPLICATIONS

This application claims the benefit of the filing date of United States Provisional Patent Application No. 63/457,885 filed on April 7, 2023, the disclosure of which is hereby incorporated by reference in its entirety.

### BACKGROUND

When hip function begins to deteriorate for a patient that has previously had a hip implant surgery, a revision hip implant surgery is generally performed to regain at least some of the lost functionality. An acetabular revision implant used for these surgeries ordinarily consists of several discreet components. One component is a metal acetabular cup shell that can be implanted with bone cement or by use of an integrated biological fixation surface. Another component is a femoral stem component that is fixed into the femoral canal. Yet another component is an articulation coupling in the form of a liner with a bearing surface that is seated in the acetabular cup and receives the femoral stem component to provide for rotation of the femoral stem component and thereby form an artificial hip j oint. An articulation couple usually has a spherical head that is fixed via a taper lock onto the proximal side of the femoral stem and a liner on the acetabular side. The liner can be fixed via a mechanical locking mechanism or through cementing into the metal shell. Additionally, the shell can be used in conjunction with bone screws that will enhance the fixation between the acetabular shell and the hip socket.

Revision hip surgery is often a complex surgical procedure where a surgeon does not have full visibility to the acetabular implant devices and functions that will be required until the patient is on the operating table. However, existing acetabular implants are not adaptable to alternative surgical approaches should a surgeon wish to change approaches intraoperatively or adaptable to patient requirements that may change postoperatively. For example, acetabular cup liners are either cemented or mechanically fixed, e.g., by bone screws or locking rings, into fully solid or porous shells. Additionally, screw hole placements in existing acetabular revision shells are limited to fixed locations in the implants even though remaining bone structure for revision surgeries may be inadequate for fixation using some of the fixed screw hole placements.

Thus, there exists a need for more adaptable acetabular shells to provide optionality during surgery and to improve surgical outcomes.

### BRIEF SUMMARY

In accordance with an aspect of the present disclosure, a shell, and an assembly with the shell, that includes a liner, for implantation into a patient, especially during an implant revision surgery, e.g., hip or shoulder revision surgeries, may be generally hemispherical. Such shells may provide more optionality during the revision surgery, better adhesion with bone cement used to fix the liner to the shell, and better fixation upon implantation of the implant into a patient. For example, the shell may have regions of varying porosity, such as certain portions having a first porosity, which may be formed by pores with consistent pore sizes, and other portions having a second porosity, which may be formed by pores with consistent pore sizes, or lack of porosity. That is, some portions may be more porous than others, and bone cement may be disposed on the more porous portions to adhere a liner to the shell. The bone cement may have better adhesion with the more porous portions of the shell and form a relatively stronger bond between the shell and the liner. In some arrangements, one or more of the porous portions may be configured to be drilled through, or in some arrangements to have a screw bored through, the one or more portions to affix the shell to bone. These one or more porous portions may be configured on the shell in shapes and sizes that are customized to the human anatomy so that the porous portions line up with bones surrounding the implantation region for passage of a bone screw through the shell and into surrounding bone.

Other examples of the shell implant may include holes already defined in the shell to receive bone screws, as well as a combination of regions or portions with varying porosities. Specifically, the shell may include solid or substantially non-porous struts extending between the rim and the peak of the dome-shaped shell to provide for strength and durability against the forces of the body when in use. The struts may be straight, curved or extending at different angles along the shell to accommodate the bone screw holes. Additionally, the shell may have porous portions between the bone screw holes and the solid struts for bone cement interdigitation. In some such arrangements, the porous portions may have a reduced thickness relative to the solid struts to provide clearance for portions of the bone cement to further enhance liner affixation.

In accordance with an aspect, an orthopedic implant may include a first surface, a second surface, and a first body portion extending between the first surface and the second surface to define a thickness of the implant, the first body portion including a first porous section defining pores, solid bounding struts including a first set of bounding struts, and a first bone screw hole extending through a thickness of the implant. Each of the struts of the first set of bounding struts may bound the first porous section. The first set of bounding struts may include a first curved strut that further bounds a portion of a perimeter of the first bone screw hole.

In some arrangements according to any of the foregoing, the first set of bounding struts may include a second curved strut bounding the first porous section.

In some arrangements according to any of the foregoing, the first curved strut and the second curved strut may be connected by a first straight strut.

In some arrangements according to any of the foregoing, the first straight strut may bound the first porous section.

In some arrangements according to any of the foregoing, the first set of bounding struts may include a second straight strut extending from the second curved strut.

In some arrangements according to any of the foregoing, the first body portion may define a curved edge spaced from the first curved strut.

In some arrangements according to any of the foregoing, the first straight strut may extend from the perimeter of the first bone screw hole.

In some arrangements according to any of the foregoing, the orthopedic implant may include a second bone screw hole extending through the thickness of the implant, wherein the first straight strut extends between the perimeter of the first bone screw hole and a perimeter of the second bone screw hole.

In some arrangements according to any of the foregoing, the first set of bounding struts may include a second straight strut extending from the first curved strut.

In some arrangements according to any of the foregoing, the orthopedic implant may include a second bone screw hole extending through the thickness of the implant, wherein the second curved strut bounds a portion of a perimeter of the second bone screw hole.

In some arrangements according to any of the foregoing, the first set of bounding struts may include a second straight strut extending from the second curved strut, the second straight strut extending from the perimeter of the second bone screw hole.

In some arrangements according to any of the foregoing, the first body portion may include a second porous section and the first set of bounding struts may include a first straight strut bounding the first porous section and the second porous section.

In some arrangements according to any of the foregoing, the first porous section may be disposed on a first side of the first straight strut and the second porous section may be disposed on a second side of the first straight strut opposite the first side.

In some arrangements according to any of the foregoing, the solid bounding struts may include a second set of struts having a second curved strut bounding the second porous section.

In some arrangements according to any of the foregoing, the second curved strut may bound a portion of the perimeter of the first bone screw hole.

In some arrangements according to any of the foregoing, the second set of struts may include the first straight strut, a third curved strut and a second straight strut bounding the second porous portion.

In some arrangements according to any of the foregoing, the first body portion may include a second bone screw hole extending through a thickness of the implant, and the third curved strut may bound a portion of a perimeter of the second bone screw hole.

In some arrangements according to any of the foregoing, the second straight strut may extend from the third curved strut and bound a third porous portion.

In some arrangements according to any of the foregoing, the solid bounding struts may include a second straight strut extending from the second curved strut and bounding the second porous section.

In some arrangements according to any of the foregoing, the first body portion may include a second porous portion and a third porous portion that each share a respective one of the first set of bounding struts with the first porous section.

In some arrangements according to any of the foregoing, the first body portion may include a second porous section and a third porous section, wherein the first porous section defines a first shape, the second porous section defines a second shape different from the first shape, and the third porous section defines a third shape different from the first and the second shapes.

In some arrangements according to any of the foregoing, the first body portion may define an elongate slot extending through the thickness of the implant.

In some arrangements according to any of the foregoing, the first surface may define a recess in the thickness of the implant adapted to receive bone cement to couple the first body portion to a liner.

In some arrangements according to any of the foregoing, the first porous section may have a thickness taken in a first direction from the first surface to the second surface that is less than a thickness of the first set of bounding struts taken in the first direction.

In some arrangements according to any of the foregoing, the first surface may be an interior surface of a dome of an acetabular shell and the second surface may be an exterior surface of the dome.

In some arrangements according to any of the foregoing, the first body portion may include a first set of solid body portions, and the first set of solid body portions and the first set of bounding struts together may bound an entirety of the first porous section.

In some arrangements according to any of the foregoing, the implant may be a ball-and-socket joint.

In some arrangements according to any of the foregoing, the implant may be a hip implant or a shoulder implant.

According to another aspect, an orthopedic implant may include a first surface, a second surface and a first body portion extending between the first surface and the second surface to define a thickness of the implant, the first body portion including a first porous portion defining a first shape, a second porous portion defining a second shape, and a third porous portion defining a third shape. The first shape may be different from the second shape and the third shape may be different from the first and second shapes. The orthopedic implant may be configured such that when the implant is implanted, the first porous portion is positioned on the first body portion to align with a first bone, the second porous portion is positioned on the first body portion to align with a second bone, and the third porous portion is positioned on the first body portion to align with a third bone.

In some arrangements according to any of the foregoing, the first bone may be an iliac bone, the second bone may be an ischial bone, and the third bone may be a pubic bone.

In some arrangements according to any of the foregoing, the first shape may be ovular.

In some arrangements according to any of the foregoing, the second shape may be trapezoidal.

In some arrangements according to any of the foregoing, the third shape may be rectangular.

### BRIEF DESCRIPTION OF THE DRAWINGS

FIG. 1 is an upper perspective view of a shell for an acetabular implant according to an embodiment of the disclosure.
FIG. 2 is a lower perspective view of the shell of FIG. 1.
FIG. 3 is a lower view of the shell of FIG. 1.
FIG. 4 is a close-up lower view of an interior of the shell of FIG. 1.
FIG. 5 is a lower view of a shell for an acetabular implant according to another embodiment of the disclosure.
FIG. 6 is a schematic view of an implanted shell of an acetabular implant according to another embodiment of the disclosure.
FIG. 7 is a schematic view of the implanted shell of FIG. 6 with a bone screw extending through the shell and an iliac bone at a first trajectory according to another embodiment of the disclosure.
FIG. 8 is a schematic view of the implanted shell of FIG. 6 with a bone screw extending through the shell and iliac bone at a second trajectory according to another embodiment of the disclosure.
FIG. 9 is a schematic view of the implanted shell of FIG. 6 with a bone screw extending through the shell and an ischial bone according to another embodiment of the disclosure.
FIG. 10 is a schematic view of the implanted shell of FIG. 6 with a bone screw extending through the shell and pubic bone according to another embodiment of the disclosure.
FIG. 11 is a lower perspective view of a shell of an acetabular implant according to another embodiment of the disclosure.
FIG. 12 is a schematic diagram of a shell of an acetabular implant according to another embodiment of the disclosure.
FIG. 13 is a lower perspective view of a shell of an acetabular implant according to another embodiment of the disclosure.
FIG. 14 is a cross-sectional view of the shell of FIG. 13.
FIG. 15 is a lower perspective view of a shell of an acetabular implant according to another embodiment of the disclosure.
FIG. 16 is a side perspective view of the shell of FIG. 15.
FIG. 17 is a schematic diagram of a shell of an acetabular implant according to another embodiment of the disclosure.
FIG. 18 is a schematic diagram of a shell of an acetabular implant according to another embodiment of the disclosure.

### DETAILED DESCRIPTION

The present disclosure describes various features and embodiments of an acetabular cup implant that may be formed, for example, by additive manufacturing processes, and that is specifically designed to enable fixation of an acetabular cup liner, or simply liner as used herein, with both a mechanical locking mechanism and bone cement that may be applied. As in the various examples below, an acetabular cup in accordance with the present disclosure includes both porous portions and solid portions. The solid portions allow for optimized support of a liner that is fixed via a locking mechanism as well as strength and rigidity, while the porous sections allow for cement interdigitation that will create an enhanced bonding surface with the liner.

Several features are shown in each of the various examples described throughout this disclosure, and it should be noted that each feature is not limited to use only with an example according to which it is described. That is, features described with respect to one example may be combined with features described in a different example or used interchangeably according to various examples where possible. As used herein, the terms "substantially, "generally," "approximately," "roughly," and "about," when used as modifiers, are intended to mean that slight deviations from absolute are included within the scope of the term so modified.

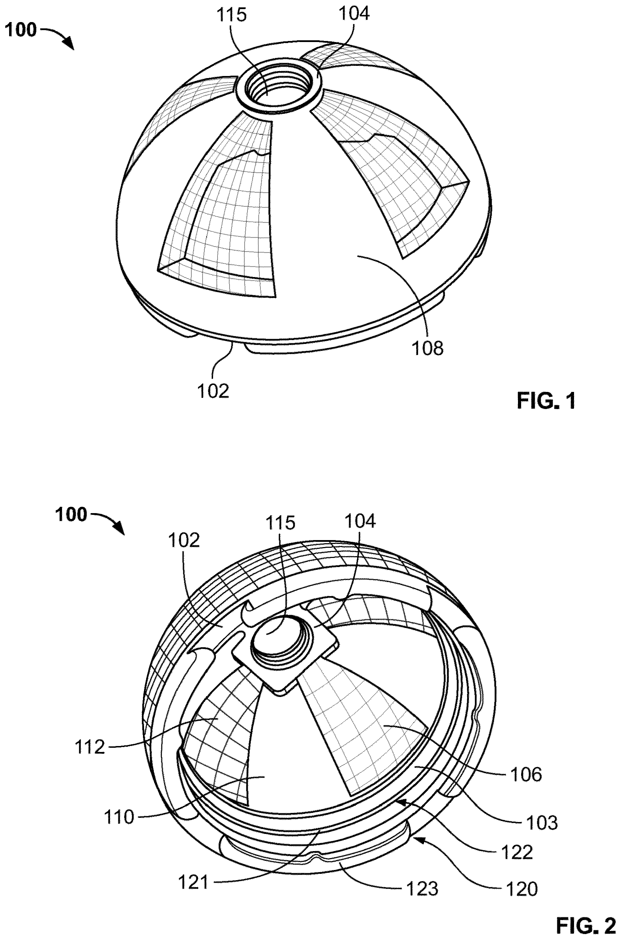

Referring now to drawings, as shown in FIGS. 1-4, shell 100 generally has a dome-shape extending from rim 102 to peak 104 and includes inner surface 106, outer surface 108, and body portion 109. Body portion 109 extends between inner and outer surfaces 106, 108 to define a thickness of the shell. Shell 100 includes solid portions 110 and porous portions 112 alternating circumferentially around inner surface 106. In some arrangements as in the example shown, porous portions 112 extend through the thickness of shell 100 defined by body portion 109. As in the example shown, the solid and porous portions 110, 112 may have generally trapezoidal shapes which allow for an even circumferential distribution of solid material and porous material at the inner region of the shell.

Porous portions 112 extend from inner lip 103 proximate rim 102 toward peak 104, terminating near opening 115 defining a peak of the shell. Porous portions 112 provide concentrated surface areas, e.g., via interconnected pores, which improve the capability of bone cement to affix a liner to inner surface 106 of shell 100. Porous regions 112 may be defined by struts such as those described in U.S. Patent No. 11,510,783 B2, U.S. Patent Appl. Publ. No. 2020/0306048 A1 and U.S. Patent Appl. Publ. No. 2020/0202043 A1, the disclosures of which are hereby incorporated herein by reference. It should be noted, however, that the bone cement will attach to any portion of the inner surface of body portion 109, including solid portions 110 or porous portions 112. As in the example shown, the set of solid and porous portions 110, 112 may be recessed relative to surrounding regions such as peak 104 and inner lip 103 to form a cement mantle. In some examples, the porous portions 112 may be recessed relative to the solid portions 110 such that the solid portions are positioned to interact with a liner locked therein while the porous portions have a gap between the inner surface of the porous portions and the liner positioned in the shell, the gap configured to receive bone cement.

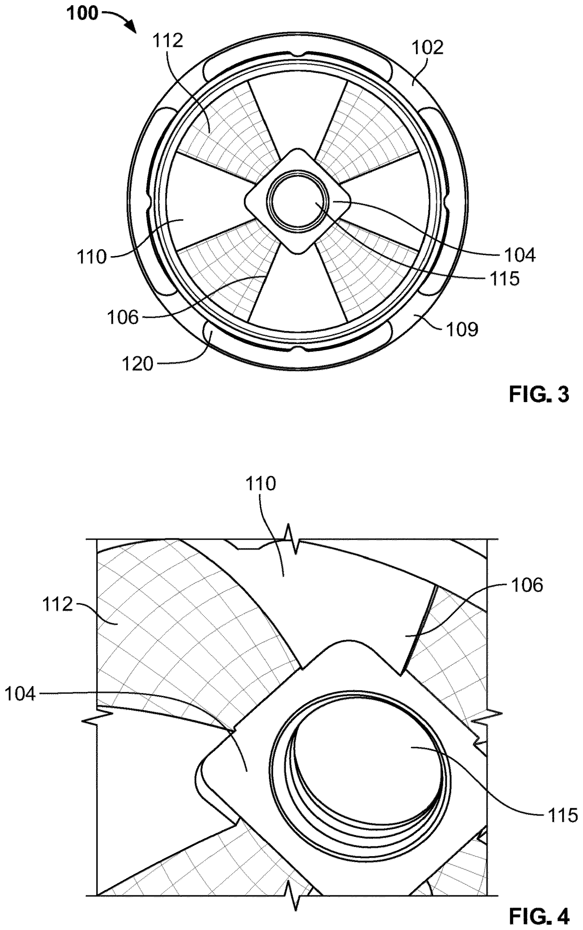

As best shown in FIGS. 2 and 3, shell 100 further includes liner locking mechanism 120 as is generally known in the art. Liner locking mechanism 120 is positioned along rim 102 and forms a tapered circumferential region 122 including a protrusion 121 and rim flanges 123 in the shell which interact with a liner to affix and maintain the liner within shell 100. As such, shell 100 allows for structural fixation of a liner via the inclusion of liner locking mechanism 120, cement interdigitation into porous portions 112 for stronger cement bonds with the shell, and a cement interface with solid regions 110 for additional cement bonding with the shell.

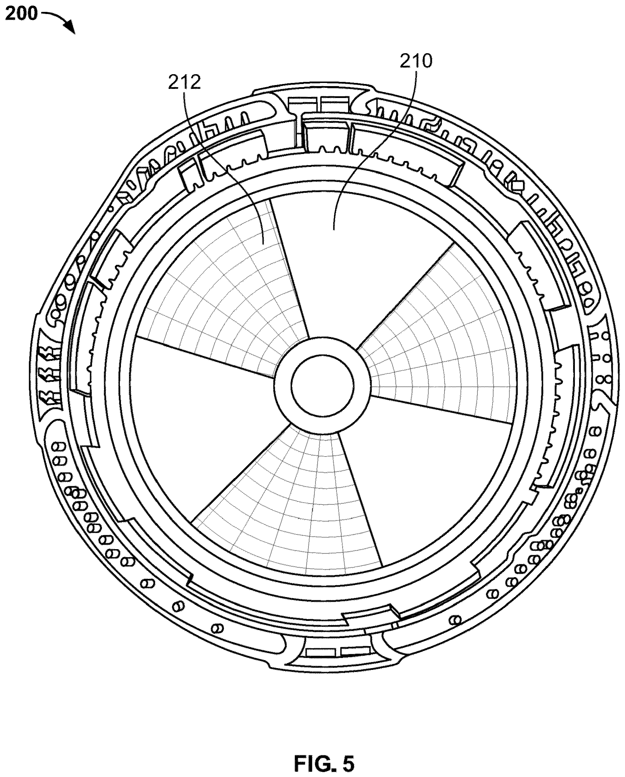

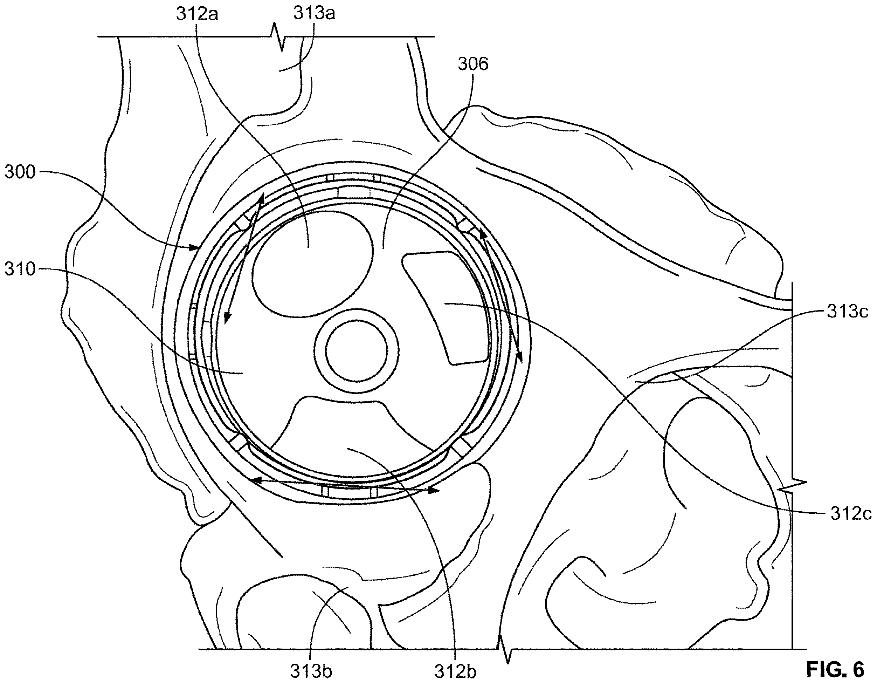

In variations of the example of FIGS. 1-4, the shapes, sizes, and number of solid portions 110 and porous portions 112 may be modified to provide for bone cement interdigitation in different locations. For instance, shell 100 in FIG. 6 is shown having four solid portions 110 alternating with four porous portions 112, whereas shell 200 is shown in FIG. 5 having three solid portions 210 alternating with three porous portions 212. In other arrangements, the shell may have any number of alternating portions. In some examples, instead of solid portions 110 being oriented vertically between rim 102 and peak 104, the solid portions may be oriented horizontally, e.g., extending circumferentially around inner surface 106. In other examples, the solid and porous portions may not be similar in size, but instead may be scattered about the shell or have irregularly shaped boundaries, as shown in examples below.

In some arrangements, porous portions 112 may be formed with either one or both repeating or randomized unit cell configurations, such as those disclosed in U.S. Patent No. 11,510,783 B2 to enhance bonding between shell 100 and bone cement applied to the porous portions. Preferably, unit cell strut lengths will fall within the range of 0.5 mm to 2.0 mm for enhanced bone cement interdigitation. Porous portions 112 may be recessed to provide relief in bone cement application areas that endure directionally dependent internal stresses.

Referring now to FIG. 6, shell 300 for an acetabular cup implant is similar to shell 100, including several features in common such as a liner locking mechanism and the combination of solid and porous portions, such that differences of shell 300 will be described herein. Shell 300 includes solid portion 310 and three distinct porous portions: iliac porous portion 312a, ischial porous portion 312b and pubic porous portion 312c. Each porous portion 312a, 312b, 312c is shaped and positioned along inner surface 306 of shell 300 to align with a corresponding bone when the shell is implanted into a patient. That is, iliac porous portion 312a corresponds to and aligns with iliac bone 313a, ischial porous portion 312b corresponds to and aligns with ischial bone 313b, and pubic porous portion 312c corresponds to and aligns with pubic bone 313c. Porous portions 312a, 312b, 312c are shaped and aligned for the purpose of drilling through these regions of shell 300 with a bone screw 314a, 314b, 314c. The shapes of each porous portion 312a, 312b, 312c as well as the trajectory that would be traveled by each bone screw 314a, 314b, 314c may be determined based on a population bone analysis, e.g., the Stryker Orthopaedic Modeling and Analytics (SOMA) database. In the illustrated example, iliac porous portion 312a is generally ovular, ischial porous portion 312b is generally trapezoidal, and pubic porous portion 312c is generally rectangular. However, porous portions 312a, 312b, 312c are not limited to such shapes. In some arrangements, porous portions 312a, 312b, 312c extend through a thickness of a body portion of shell 300.

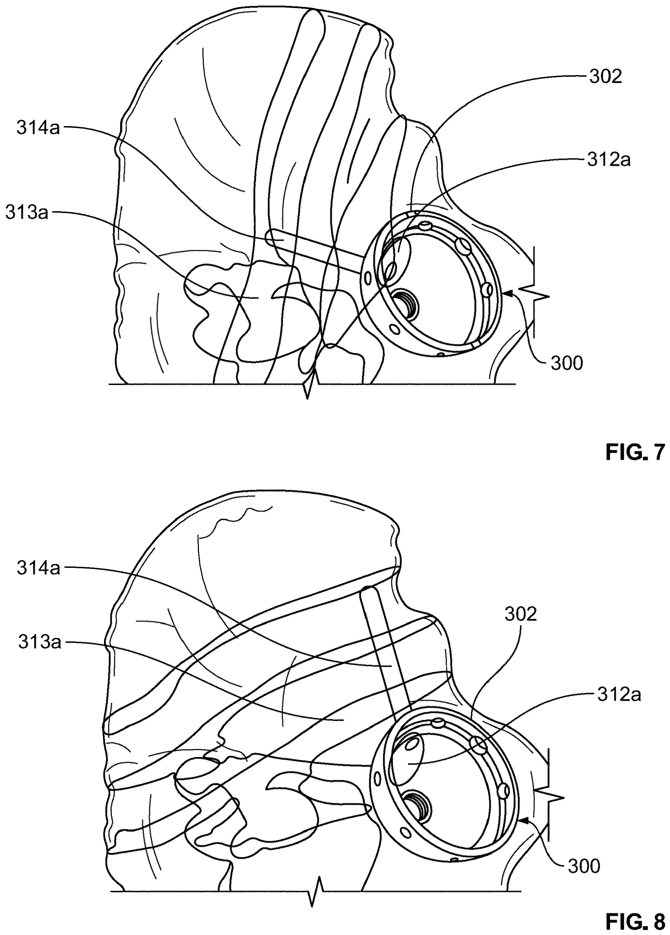

With reference to FIGS. 7-10, each of porous portions 312a, 312b, 312c of shell 300 are shaped such that bone screws of about 40-60mm in length may be passed through areas within each shape at a range of angular trajectories of +/- 5 to 20 degrees along a preferential direction. Bone screw lengths and angular trajectories may be refined based on a population bone analysis. A population bone analysis may be performed by generating cross-sections of the three bony targets (*e.g.,* iliac column, ischial column and pubic column) of a representative pelvic model at progressive increments of depth into the bone and away from shell 300. Thereafter, the collection of cross-sectional shapes may be viewed as an overlay, and the least common overlay zone between the cross-sections may be used to approximate the respective shape of the porous zone within shell 300. Such a starting position of porous shapes would allow a range of screw lengths at slightly varying trajectories to be able to penetrate into the corresponding bone while at the same time avoiding violation of the relevant bony boundaries. For example, bone screw 314a may be passed through iliac bone 313a and through one location of iliac porous portion 312a of implanted shell 300 at one trajectory, as shown in FIG. 7, and through another location of the iliac porous portion at another trajectory, as shown in FIG. 8. For iliac bone 313a, the shape of iliac porous portion 312a is roughly oval with the major axis of the oval spanning radially outwards, ending either slightly inferior to rim 302, as shown in FIGS. 7 and 8, or ending at the superior boundary of the taper of the shell. This shape allows bone screws of up to 60 mm in length to be positioned within the iliac cross-sections with +/-20 degrees or radial trajectory variation.

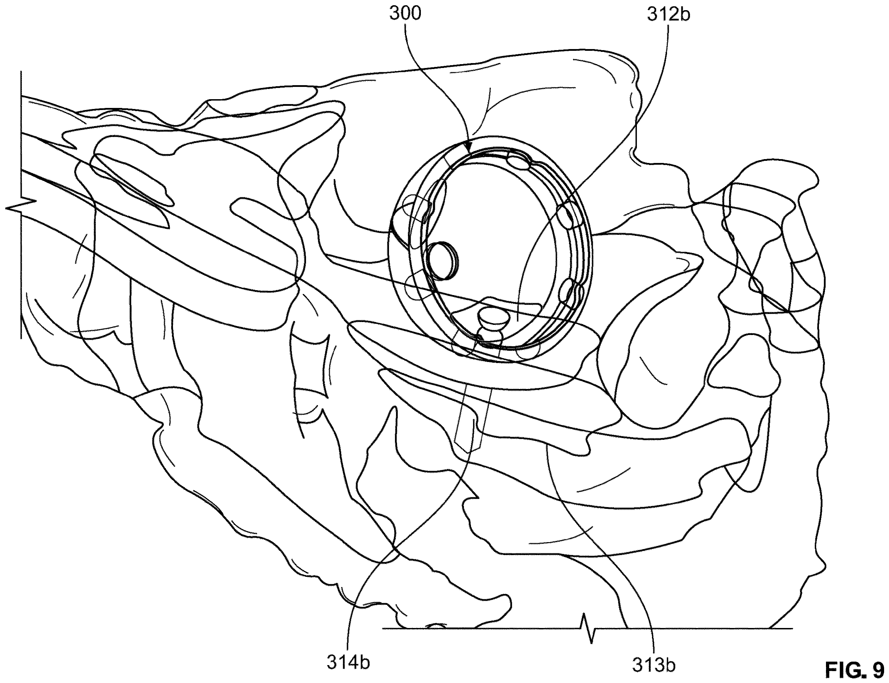

As shown in FIG. 9, bone screw 314b may be passed through ischial bone 313b. The shape of ischial porous portion 312b is roughly trapezoidal, with the longer arm or diagonal of the trapezoid spanning circumferentially, either slightly superior (i.e., shallower, in reference to the patient's anatomy, when the shell is properly seated in a patient's acetabulum) to the taper of shell 300, as shown, or overlapping onto the taper. This shape allows bone screws of up to 40mm in length to be positioned within the ischial cross-sections with +/- 10 degrees of circumferential trajectory variation.

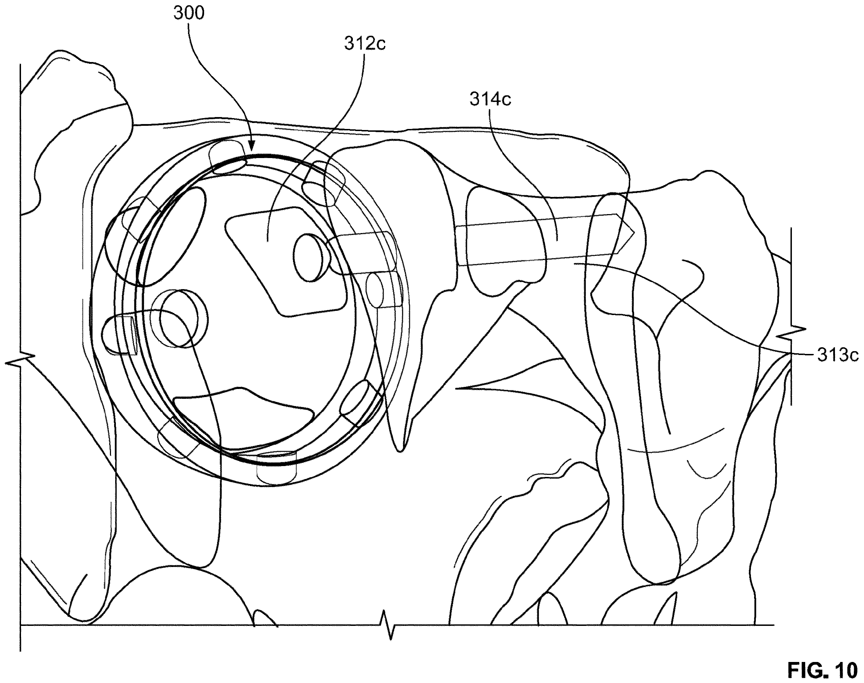

As shown in FIG. 10, bone screw 314c may be passed through pubic bone 313c. The shape of pubic porous portion 312c is roughly rectangular, with the longer diagonal of the rectangle spanning circumferentially, either slightly posterior (i.e., deeper, in reference to the patient's anatomy, when the shell is properly seated in a patient's acetabulum) to the taper of shell 300, as shown, or overlapping onto the taper. This shape allows bone screws of up to 60mm in length to be centered within the pubic cross-sections with +/- 5 degrees of circumferential trajectory variation.

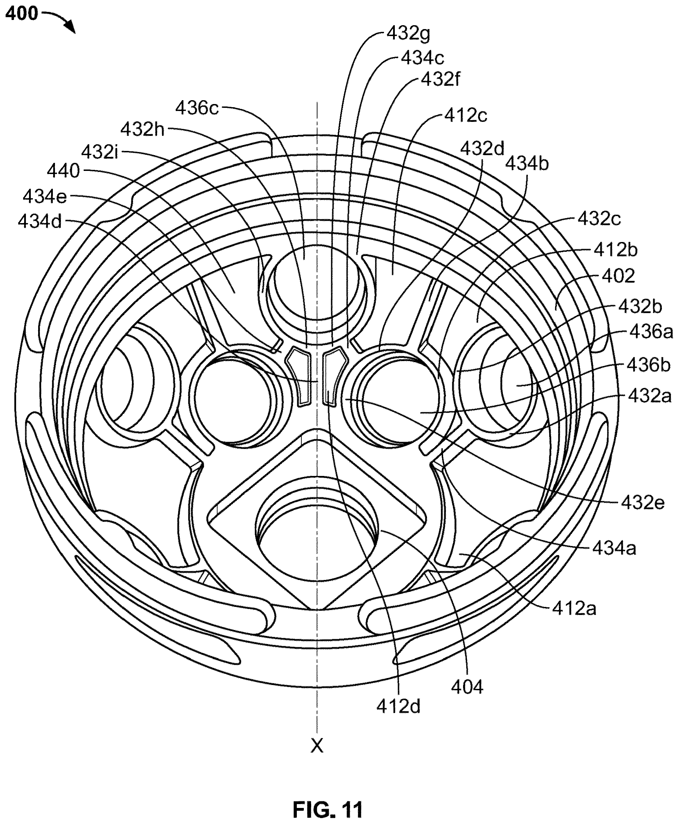

Referring now to the example of FIG. 11, shell 400 for an acetabular cup implant is similar to shell 100 described above, including common features such as the combination of both solid and porous portions, such that differences of shell 400 will be described herein. Shell 400 includes several porous portions, the following of which are visible in the view shown: first porous portion 412a, second porous portion 412b, third porous portion 412c, and fourth porous portion 412d. Shell 400 further includes a plurality of solid struts, which as in the example shown may be in the form of supports, separating the porous portions. It is noted that not all solid struts are shown in the view of FIG. 11, and thus the example shown in FIG. 11 is not limited to the struts identified herein. Included among the solid struts are curved struts, such as first curved strut 432a, second curved strut 432b, third curved strut 432c, fourth curved strut 432d, fifth curved strut 432e, sixth curved strut 432f, seventh curved strut 432g, eighth curved strut 432h and ninth curved strut 432i. Additionally included among the plurality of solid struts are first straight strut 434a, second straight strut 434b, third straight strut 434c, and fourth straight strut 434d. Shell 400 further defines several bone screw holes extending through the thickness of the shell, including first bone screw hole 436a, second bone screw hole 436b, and third bone screw hole 436c.

First curved strut 432a extends vertically from a solid edge portion proximate rim 402 toward peak 404 and is coupled to second curved strut 432b and first straight strut 434a. First straight strut 434a extends vertically from an intersection of first and second curved struts 432a, 432b toward peak 404. First and second curved struts 432a, 432b mirror one another and bound first bone screw hole 436a. First curved strut 432a and first straight strut 434a bound a portion of first porous portion 412a.

Second straight strut 434b extends vertically from the solid edge portion proximate rim 402 toward peak 404 and is coupled to third curved strut 432c and fourth curved strut 432d. Third and fourth curved struts 432c, 432d extend generally vertically from second straight strut 434b toward peak 404. Fifth curved strut 432e extends generally vertically from fourth curved strut 432d toward peak 404. Fourth curved strut 432d and fifth curved strut 432e are coupled at their intersection to third straight strut 434c. The combination of fourth and fifth curved struts 432d, 432e mirrors third curved strut 432c, and these three curved struts bound second screw hole 436b. Second porous portion 412b is bound by second curved strut 432b, first straight strut 434a, third curved strut 432c, second straight strut 434b and a solid edge portion proximate rim 402.

Sixth curved strut 432f extends generally vertically from the solid edge portion proximate rim 402 toward peak 404. Sixth curved strut 432f is coupled to third straight strut 434c and seventh curved strut 432g at their intersection. Ninth curved strut 432i extends generally vertically from the solid edge portion proximate rim 402 toward peak 404. Ninth curved strut 432i is coupled to fifth straight strut 434e and eighth curved strut 432h. Eighth curved strut 432h is coupled to fourth straight strut 434d and seventh curved strut 432g at their intersection. The combination of sixth curved strut 432f and seventh curved strut 432g mirrors the combination of eight curved strut 432h and ninth curved strut 432i, and third bone screw hole 436c is bound by the sixth, seventh, eighth and ninth curved struts. Further, third porous portion 412c is bound by second straight strut 434b, fourth curved strut 432d, third straight strut 434c, sixth curved strut 432f and the solid edge portion proximate rim 402. Fourth porous portion 412d is bound by fifth curved strut 432e, third straight strut 434c, seventh curved strut 432g and fourth straight strut 434d.

It should be noted that shell 400 includes additional porous portions, solid struts and bone screw holes beyond what is described above. However, in FIG. 11, along axis X drawn collinear with fourth straight strut 434d (located approximately down the middle of shell 400 as shown in FIG. 11), a mirror image is formed on either side of the axis. Thus, the mirrored side includes strut, porous portion, and bone screw hole features substantially similar to those described above. One exception in the illustrated example is that instead of having a porous portion on the side of shell 400 mirroring third porous portion 412c, the portion opposing the third porous portion defines a window 440. Window 440 is an opening extending through the thickness of shell 400 and provides for an additional opening preferably for receiving a bone screw therethrough for further fixation of the shell in a unitized cup-cage construct for receiving a protrusion of an acetabular reconstruction cage in a cup-cage construct.

The configuration of shell 400, along with any other embodiment (e.g., those like the examples of shells 100, 200) of the shell implementing porous portions, derives several benefits from the combination of solid and porous portions. The porous portions, e.g., porous portions 120, 220, 412a-412d, are positioned along shell 400 to correspond with anatomically relevant parts of patients and to provide for enhanced cement interdigitation. Meanwhile, solid struts, e.g., solid struts 432a-432i and 434a-434h, and solid portions, e.g., solid portions 110 and 210 are incorporated into shells such as shell 400 to maintain the structural integrity of portions of the shell, e.g., bone screw holes 436a-436c, and the shell as a whole. The solid struts are also used to modulate stiffness in order to direct the deformation direction of the shell under a press fit between the anterior and posterior columns of the acetabulum.

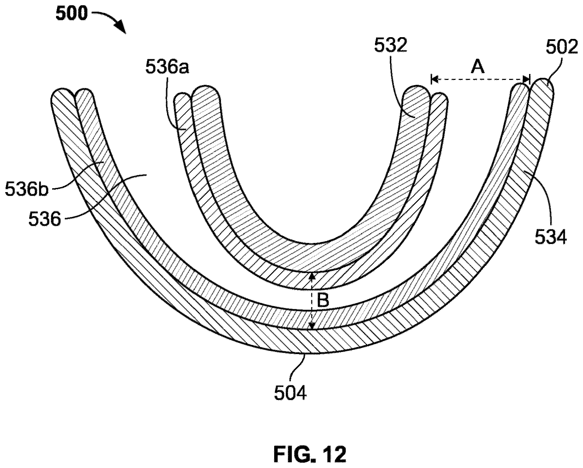

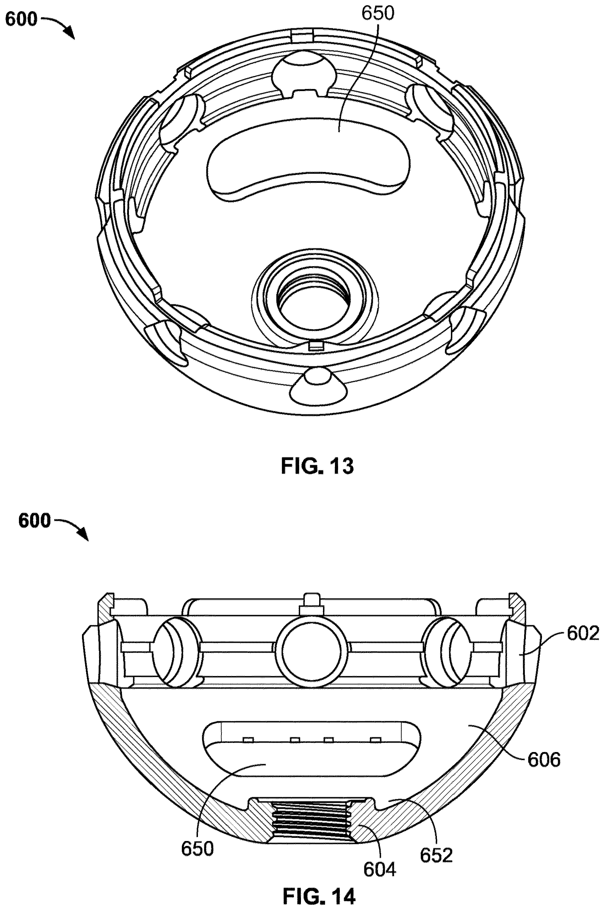

With reference to FIG. 12, shell 500 for an acetabular cup implant includes inner porous layer 532 configured with pores for cement interdigitation and outer porous layer 534 configured with pores for bone ingrowth and a surface having sufficient surface roughness to provide friction for grip. Shell 500 further includes intermediate layer 536 interposed between inner porous layer 532 and outer porous layer 534, which may be solid or porous and, when porous, may have a gradient porosity. Intermediate layer 536 extends between inner solid boundary 536a, which abuts inner porous layer 532, and outer solid boundary 536b, which abuts outer porous layer 534. In such configurations, which may be used in conjunction with embodiments like those of the examples of shells 100, 200, 300, 400, intermediate layer 536, especially when full solid, provides additional support to shell 500 and modulates the stiffness of the shell while inner and outer porous layers 532, 534 provide their above-described bone cement and bone interactions at the surfaces of the shell. The thickness of intermediate layer 536 (provided by the distance between inner solid boundary 536a and outer solid boundary 536b) may vary throughout the cross-section of shell 500, as shown in FIG. 14. For example, solid layer 536 defines a first thickness "A" proximate rim 502 of shell 500 and a second thickness "B" proximate peak 504 of the shell less than first thickness "A." This difference in thickness promotes a secure press fit between the anterior and posterior acetabular columns while still maintaining the utility of porous regions in inner and outer porous portions 532, 534 for cement interdigitation and bony ingrowth, respectively, in shell 500. As shown, the thickness of shell 500 decreases gradually from the region proximate rim 502 to the region proximate peak 504.

As shown in FIGS. 13 and 14, shell 600 for an acetabular cup implant defines elongated slot 650 extending generally horizontally around a circumference of the shell. Elongated slot 650 is an opening extending through the thickness of shell 600 and adapted to receive a bone screw therethrough. Elongated slot 650 provides for additional target space through which a bone screw can be inserted along shell 600, *e.g.,* a greater number of insertion location options as compared to a circular bone screw hole and therefore requires less precision and provides additional trajectory options for insertion of a bone screw. Shell 600 further defines pocket 652 as shown in FIG. 14, which is a recessed portion of the shell along inner surface 606 between rim 602 and peak 604. Pocket 652 can interact with and be filled with bone cement. Pocket 650, particularly in combination with a liner locking mechanism as described above, may allow a surgeon the intraoperative flexibility to cement in a liner or lock the liner in shell 600. In some arrangements, pocket 650 may be recessed at a depth of approximately 2mm. In some examples, rather than elongated slot 650 being an opening extending through the thickness of shell 600, the slot may be a fully porous recessed portion having the shape of the elongate slot which is adapted to be drilled through with bone screws.

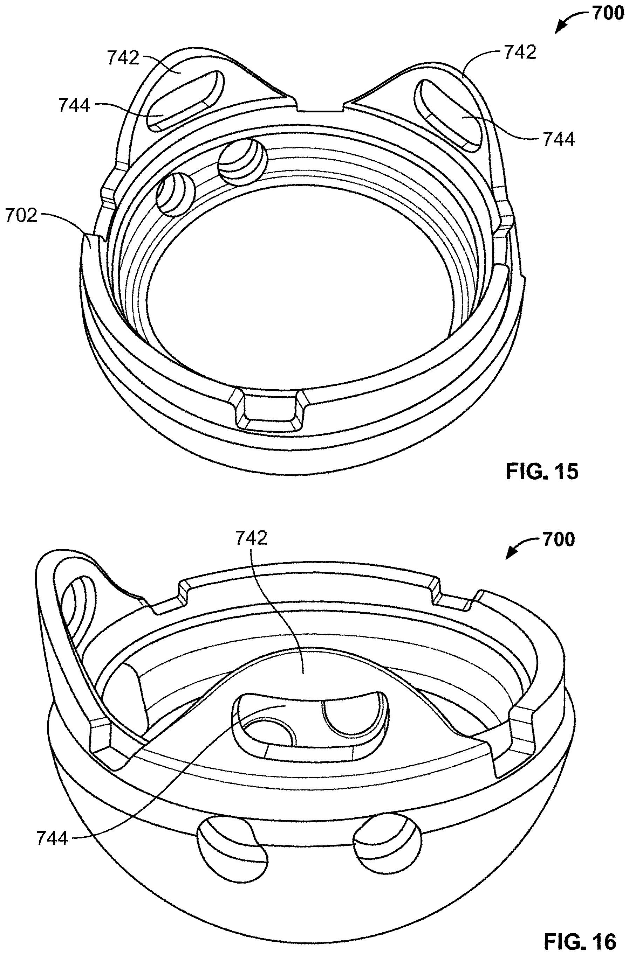

Referring now to FIGS. 15 and 16, shell 700 for an acetabular cup includes ears 742 projecting from rim 702 of the shell such that the ears extend beyond the bounds of liner locking mechanism 720. Each ear 742 includes an opening 744 extending through a thickness of the ear which may be sized and shaped to receive a bone screw for fixation of shell 700 to corresponding bone when implanted in a patient. Ears 742 may be utilized to facilitate appropriate placement of shell 700 and entire acetabular implant when being implanted, while still maintaining adequate fixation. Ears like those of ears 742 may be used in conjunction with any one or any combination of the embodiments contemplated by the present disclosure, including embodiments like those of the examples of shells 100, 200, 300, 400.

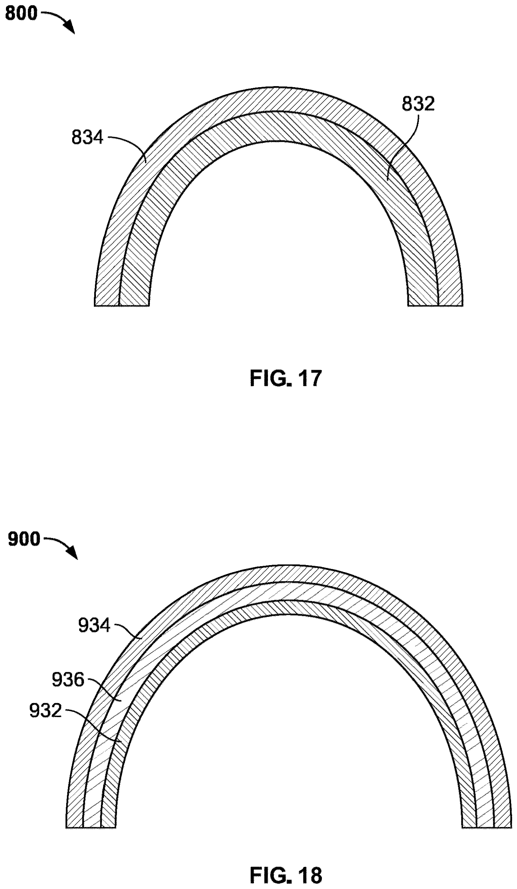

The shell for an acetabular cup implant in accordance with any of the embodiments described herein may be additively manufactured using any of the techniques described throughout U.S. PatentNo. 11,510,783 B2, U.S. Patent Appl. Publ. No. 2020/0306048 A1 and U.S. Patent Appl. Publ. No. 2020/0202043 A1. Additive manufacturing allows for the production of shells and implants corresponding to computer-aided design (CAD) models and having transitional, e.g., gradient, regions between solid and porous regions or porous-porous cross-sectional regions defined by overlapping porous configurations having a greater density than the porous configurations would have separately, but still a greater porosity than a solid structure, in which such cross-sectional regions may correspond to overlapping CAD models of the individual porous configurations. For instance, in one example, first shell cross-section 800 shown in FIG. 17 includes an outer porous layer 834 and an inner porous layer 832. Outer porous layer 834 is beneficial for osseo-integration, and inner porous layer 832 is beneficial for bone cement interdigitation. Due to the capabilities of additive manufacturing, outer porous layer 834 may transition either smoothly in a gradient fashion or abruptly to inner porous layer 832.

In another example, second shell cross-section 900 shown in FIG. 18 similarly includes an outer porous layer 934 and an inner porous layer 932 as described with respect to first shell cross-section 800. However, second shell cross-section 900 additionally includes a center layer 936 interposed between outer porous layer 934 and inner porous layer 932. Center layer 936 may be, *e.g.,* a solid layer, a structural strut layer, a layer of gradient porosity, or a pseudo-solid layer, and may be "sandwiched" between outer porous layer 934 and inner porous layer 932 to prevent bone cement from entering the osseo-integrative outer porous layer while also posing advantages for structural stability and stiffness control. The configurations of FIGS. 19 and 20 are further described in U.S. Pat. Appl. Publ. No. 2020/0306048 A1. Such configurations may be used in conjunction with any one or any combination of the embodiments contemplated by the present disclosure, including embodiments like those of the examples of shells 100, 200, 300, 400, 500.

In alternative arrangements, some or all of the recited solid portions may be replaced by porous portions with a different porosity, whether lesser or grater, than the recited porous portions.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. An orthopedic implant (100, 400), comprising:
a first surface (106);
a second surface (108); and
a first body portion (109) extending between the first surface (106) and the second surface (108) to define a thickness of the implant (100, 400), the first body portion (109) including a first porous section (112, 412a-412c) defining pores, solid bounding struts including a first set of bounding struts (432a-432i, 434a-434d), and a first bone screw hole (436a-436c) extending through a thickness of the implant,
wherein each of the struts of the first set of bounding struts (432a-432i, 434a-434d) bound the first porous section (412a-412c), and
wherein the first set of bounding struts (432a-432i, 434a-434d) includes a first curved strut (432a-432i) that further bounds a portion of a perimeter of the first bone screw hole (436a-436c).

2. The orthopedic implant (100, 400) of claim 1, wherein the first set of bounding struts (432a-432i, 434a-434d) includes a second curved strut (432a-432i) bounding the first porous section (412a-412c).

3. The orthopedic implant (100, 400) of claim 2, wherein the first curved strut (432a-432i) and the second curved strut (432a-432i) are connected by a first straight strut (434a-434d).

4. The orthopedic implant (100, 400) of claim 3, wherein the first straight strut (434a-434d) bounds the first porous section (412a-412c).

5. The orthopedic implant (100, 400) of claim 3, wherein the first set of bounding struts (432a-432i, 434a-434d) includes a second straight strut (434a-434d) extending from the second curved strut (432a-432i).

6. The orthopedic implant (100, 400) of claim 4 or claim 5, wherein the first body portion (109) defines a curved edge spaced from the first curved strut (432a-432i).

7. The orthopedic implant (100, 400) of any one of claims 3-6, wherein the first straight strut (434a-434d) extends from the perimeter of the first bone screw hole (436a-436c).

8. The orthopedic implant (100, 400) of claim 7, further comprising a second bone screw hole (436a-436c) extending through the thickness of the implant (100, 400), wherein the first straight strut (434a-434d) extends between the perimeter of the first bone screw hole (436a-436c) and a perimeter of the second bone screw hole (436a-436c).

9. The orthopedic implant (100, 400) of any one of claims 3-8, wherein the first set of bounding struts (432a-432i, 434a-434d) includes a second straight strut (434a-434d) extending from the first curved strut (432a-432i).

10. The orthopedic implant (100, 400) of any one of claims 3-9, further comprising a second bone screw hole (436a-436c) extending through the thickness of the implant (100, 400), wherein the second curved strut (432a-432i) bounds a portion of a perimeter of the second bone screw hole (436a-436c).

11. The orthopedic implant (100, 400) of claim 10, wherein the first set of bounding struts (432a-432i, 434a-434d) includes a second straight strut (434a-434d) extending from the second curved strut (432a-432i), the second straight strut (434a-434d) extending from the perimeter of the second bone screw hole (436a-436c).

12. The orthopedic implant (100, 400) of any one of claims 1-11, wherein the first body portion (109) includes a second porous section (412a-412c) and the first set of bounding struts (432a-432i, 434a-434d) includes a first straight strut (434a-434d) bounding the first porous section (412a-412c) and the second porous section (412a-412c).

13. The orthopedic implant (100, 400) of claim 12, wherein the first porous section (412a-412c) is disposed on a first side of the first straight strut (434a-434d) and the second porous section (412a-412c) is disposed on a second side of the first straight strut (434a-434d) opposite the first side.

14. The orthopedic implant (100, 400) of claim 12 or claim 13, wherein the solid bounding struts (432a-432i, 434a-434d) include a second set of struts (432a-432i, 434a-434d) having a second curved strut (432a-432i) bounding the second porous section (412a-412c).

15. The orthopedic implant (100, 400) of claim 14, wherein the second curved strut (432a-432i) bounds a portion of the perimeter of the first bone screw hole (436a-436c).
